# EUROPEAN PATENT SPECIFICATION

(11) **EP 3 661 590 B1**
(45) Date of publication and mention of the grant of the patent: **01.09.2021**
(21) Application number: 18755375.5
(22) Date of filing: 31.07.2018
(51) Int. Cl.: A61N 1/05, A61B 5/00, A61N 1/36, A61B 5/024, A61B 5/08, A61B 5/145

(54) **AURICULAR STIMULATION DEVICE**
VORRICHTUNG ZUR AURIKULÄREN STIMULATION
DISPOSITIF DE STIMULATION AURICULAIRE

(30) Priority: 31.07.2017 US 201762539178 P
(43) Date of publication of application: 10.06.2020
(73) Proprietor: The Feinstein Institute for Medical Research, Manhasset, NY 11030 (US)
(72) Inventor: BOUTON, Chad E., Darien Connecticut 06820 (US); ZANOS, Theodoros P., Astoria New York 11102 (US); DATA-CHAUDHURI, Timir B., Great Neck New York 11021 (US); MARTIN, Andrew, Bayport New York 11705 (US); MONTALBANO, Christopher, Huntington New York 11743 (US); MONTALBANO, Gregory, Huntington New York 11743 (US); BEAMER, Phillip, Northport New York 11768 (US)
(74) Representative: Schrell, Andreas
(86) International application number: PCT/US2018/044568
(87) International publication number: WO 2019/028000

(56) References cited:
- WO-A1-2015/192114
- WO-A1-2017/120023
- US-A1- 2017 043 160
- US-B1- 9 415 220

## Description

### TECHNICAL FIELD

The present disclosure relates to treatment devices, and diagnostic and therapeutic systems and, more specifically, to auricular stimulation devices, diagnostic and therapeutic systems.

### BACKGROUND

The auricular branch of the vagus nerve (also known as Alderman's nerve or Arnold's nerve) is located in the ear and supplies sensory innervation to the skin of the ear canal, tragus, and auricle. The auricular branch reaches the surface of the ear and divides into two branches. The first joins the posterior auricular nerve and the second is distributed to the skin on the back of the ear or auricle and to the posterior part of the ear canal.

Stimulation of the auricular branch of the vagus nerve has been shown to have diagnostic and therapeutic benefits. For example, various studies have shown that stimulation of the auricular branch of the vagus nerve can be used to treat seizures, atrial fibrillation, depression, diabetes, endotoxemia, myocardial infarction, and tinnitus (see references cited in the appendix). The related prior art is disclosed by WO2017120023.

In view of at least the foregoing benefits of stimulating the auricular branch of the vagus nerve, there is a continuing need for more effective auricular stimulation devices.

### SUMMARY

The invention is defined in the appended claims. Aspects, embodiments and examples disclosed herein which do not fall within the scope of the appended claims do not form part of the invention, and are merely provided for illustrative purposes.

### BRIEF DESCRIPTION OF THE DRAWINGS

Various aspects of the present disclosure are described hereinbelow with reference to the drawings, which are incorporated in and constitute a part of this specification, wherein:
FIG. 1A is a perspective view of one embodiment of a stimulation device configured to stimulate the auricular branch of the vagus nerve in accordance with the present disclosure, the stimulation device including an outer probe, the outer probe illustrated in an unbent configuration;
FIG. 1B is a perspective view of the stimulation device of FIG. 1A with the outer probe thereof illustrated in a bent configuration;
FIG. 2 is a perspective view, with parts separated, of the stimulation device of FIGS. 1A and 1B;
FIG. 3 is a cross-sectional view taken along section line 3-3 of FIG. 1B;
FIG. 4 is a cross-sectional view taken along section line 4-4 of FIG. 1B;
FIG. 5 is a schematic view of a system configured to stimulate the auricular branch of the vagus nerve in accordance with the present disclosure;
FIG. 6 is a flowchart of an exemplary method of diagnosing and treating a condition with a stimulation device configured to stimulate the auricular branch of the vagus nerve in accordance with the present disclosure;
FIG. 7 is a flowchart of an exemplary treatment method of stimulating an auricular branch of the vagus nerve, such as with the stimulation device of FIGS. 1A and 1B, in accordance with the present disclosure; and
FIG. 8 is a perspective view of another embodiment of a stimulation device configured to stimulate the auricular branch of the vagus nerve in accordance with the present disclosure.

### DETAILED DESCRIPTION

Embodiments of the present disclosure are now described in detail with reference to the drawings in which like reference numerals designate identical or corresponding elements in each of the several views. As used herein, the term "clinician" refers to a doctor, a nurse, or any other care provider and may include support personnel. As used herein, the term "electrode" is defined herein as a single electrode or an array of electrodes.

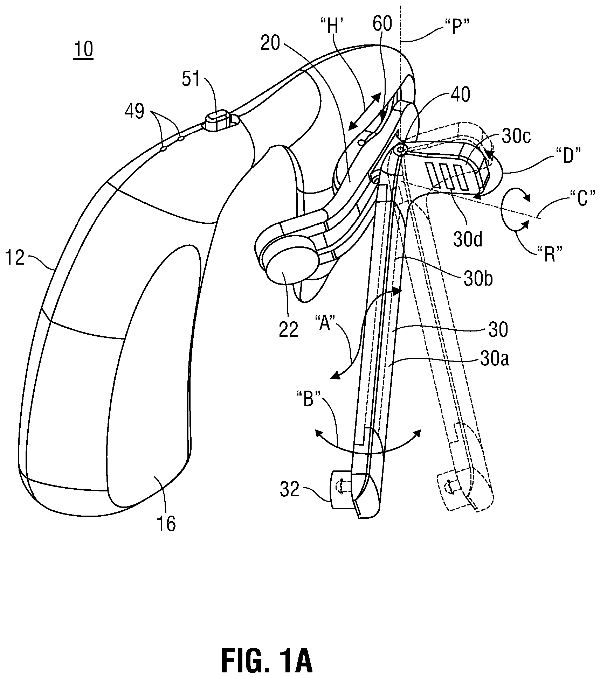

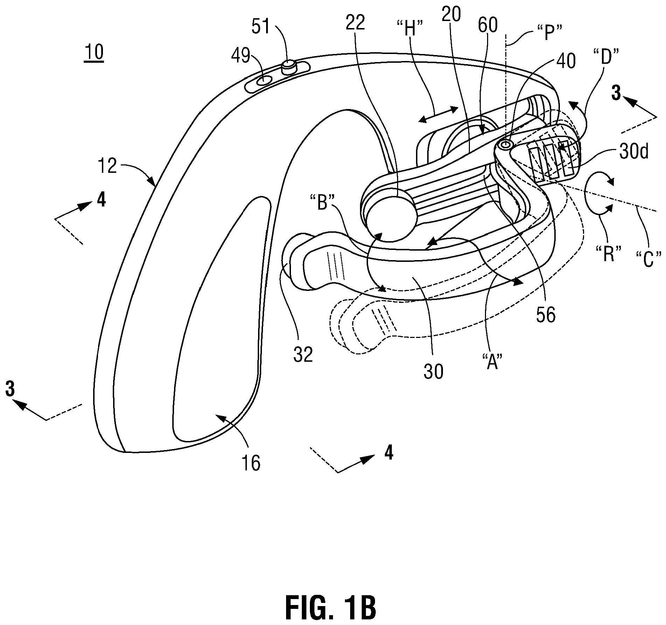

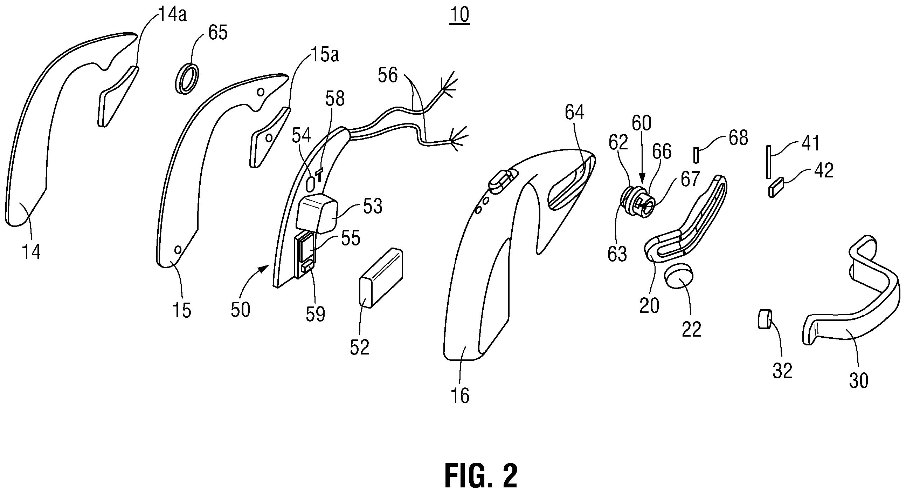

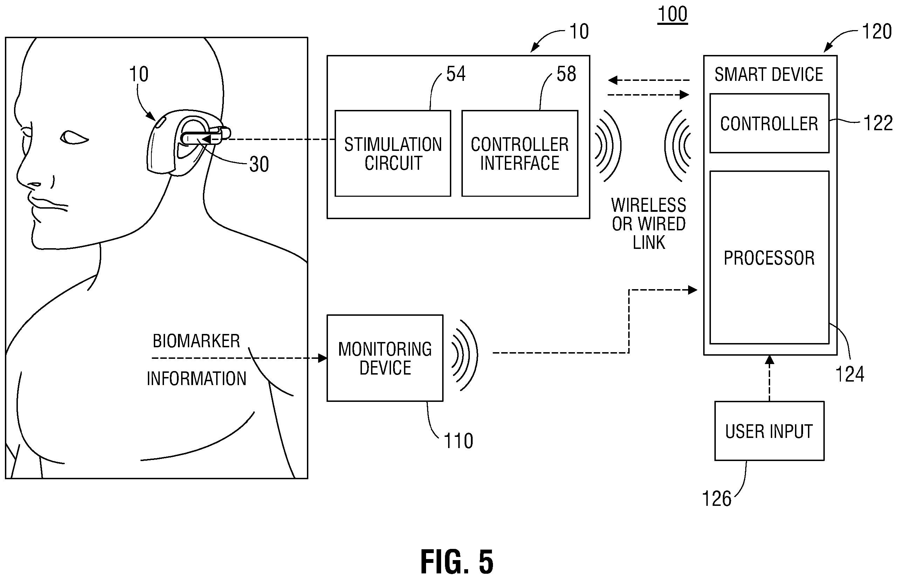

Referring now to FIGS. 1A and 1B, one embodiment of a StimClip™ or auricular stimulation device 10 is shown in accordance with the present disclosure. The stimulation device 10 includes a housing 12, a first or inner arm 20 (e.g., a probe), and a second or outer arm 30 (e.g., a probe). The housing 12 is shaped and configured for being secured over the ear of a patient (see FIG. 5). As seen in FIG. 2, the housing 12 of stimulation device 10 includes an outer flat portion 14, a middle flat portion 15, and an outer shell-shaped portion 16. Two triangular spacers 14A and 15A are provided in proximity to outer flat portion 14 and middle flat portion 15 to prevent housing portions 14 and 15 from abutting against each other.

With continued reference to FIGS. 1A-3, the housing 12 of stimulation device 10 supports an adjustment mechanism 60 for horizontally and rotationally adjusting the position of the probes 20, 30 for fitting or conforming the stimulation device 10 to ears having a variety of sizes and shapes. In particular, as indicated by arrows "H," adjustment mechanism 60 is slidably movable relative to housing 12 through mounting slot 64 of housing 12 to selectively axially move probes 20, 30 relative to housing 12. Further, adjustment mechanism 60 defines a central axis "C" therethrough about which probes 20, 30 selectively rotate relative to housing 12 and adjustment mechanism 60, as indicated by arrows "R." The stimulation device 10 is configured to be disposed on the left ear of a patient; however, in embodiments, a stimulation device 10 may be configured (e.g., as a mirror image of the left ear configuration) to be disposed on the right ear of the patient either separately or in conjunction with a stimulation device 10 disposed on the left ear of a patient. In addition, the stimulation device 10 may come in a variety of sizes to be disposed on the ear of an infant, a child, and/or an adult. The adjustment mechanism 60 also secures the inner and outer probes 20, 30 to the housing 12 and in a fixed position relative to housing 12 upon tightening of a mounting nut 65 of adjustment mechanism 60.

In particular, the adjustment mechanism 60 of the stimulation device 10 includes a mounting shaft 62 that is selectively slidably secured, as indicated by arrows "H," and selectively rotatably secured, as indicated by arrows "R," in a mounting slot 64 defined by the outer shell-shaped portion 16 of the housing 12. Allowing the mounting shaft 62 to selectively slide along and/or selectively rotate within the mounting slot 64 enables the housing 12 and the probes 20, 30 of the stimulation device 10 to be fitted or conformed to ears having a variety of sizes and shapes. As detailed herein, rotation of the probes 20, 30 relative to housing 12 is limited by movement of pin 68 through a predetermined arc length defined by opposite ends of pin slot 67. Once a suitable size and/or comfort is established mounting nut 65 can be threadably rotated or tightened on mounting shaft 62 of adjustment mechanism 60 to fix the adjustment mechanism 60 and the probes 20, 30 relative to the housing 12.

The mounting shaft 62 of adjustment mechanism 60 includes an inner segment 63 that extends inward and receives a mounting nut 65 thereabout to secure the mounting shaft 62 of adjustment mechanism 60 within the mounting slot 64 of the housing 12. The mounting shaft 62 also includes outer segment 66 that extends outward and defines a pin slot 67 that receives a pin 68 to secure the inner probe 20 to the outer segment 66 of the mounting shaft 62. Pin slot 67 is configured to limit rotational movement of the inner and outer probes 20, 30 relative to housing 12 such that pin 68 is configured to abut opposite ends of pin slot 67 to prevent further rotational movement of the probes 20, 30 in a given direction.

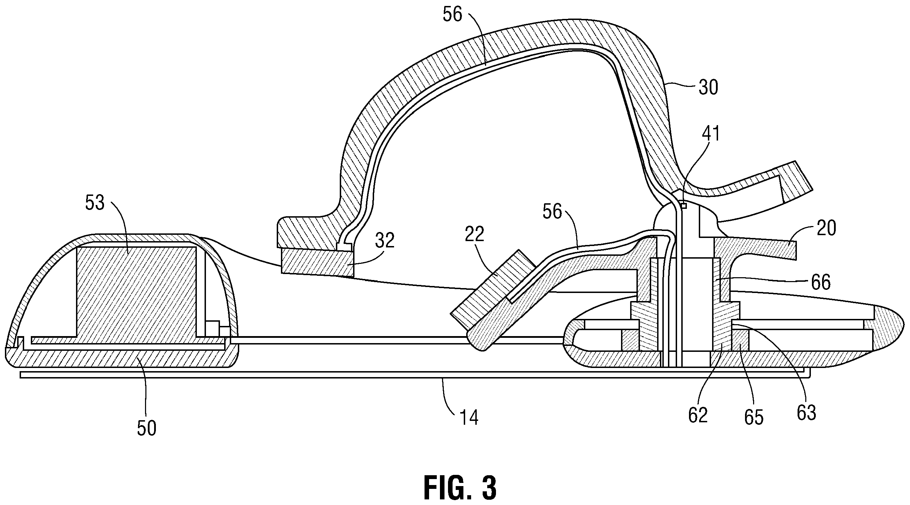

With continued reference to FIGS. 2 and 3, the inner and outer probes 20, 30 of the stimulation device 10 are pivotally mounted to one another by a pivot 40 of the stimulation device 10 that defines pivot axis "P." The pivot 40 includes a pivot pin 41 and a biasing member 42 that urges the inner and outer probes 20, 30 towards one another and into a tissue clamping position as shown in FIG. 3. The biasing member 42 may be any suitable spring (e.g., a torsion spring) disposed about the pivot pin 41.

The inner probe 20 of the stimulation device 10 includes a first or inner electrode 22. The outer probe 30 of stimulation device 10 includes a second or outer electrode 32 that opposes the inner electrode 22 and is offset from the inner electrode 22.

The outer probe 30 of the stimulation device 10 includes an elongate body 30a that is formed of a flexible material and supports one or more flexible wires 30b that extend through elongate body 30a to facilitate flexing and/or bending of elongate body 30a, as indicated by arrows "A," between an unbent configuration (FIG. 1A) and one or more bent configurations (FIG. 1B). The elongate body 30a and/or flexible wires 30b of outer probe 30 may be formed of any suitable polymeric and/or metallic material. While outer probe 30 may be bent in any number of configurations to accommodate different user comforts, ear sizes, ear shapes, etc., flexible wires 30b are configured to maintain outer probe 30 fixed in a respective configuration until subsequently bent to a different configuration. The outer probe 30 further includes a rigid foot 30c that extends transverse to the elongate body 30a of the outer probe 30. The foot 30c may include a finger grip 30d that may include any suitable surface texturing such as ridges, knurling, etc. The foot 30c is actuatable a user's finger, as indicated by arrows "D," to facilitate pivotal movement of outer probe 30 relative to inner probe 20 about pivot axis "P."

The biasing member 42 of pivot 40 urges the inner and outer probes 20, 30 towards one another, such that the inner and outer electrodes 22, 32 of the inner and outer probes 20, 30, respectively, are urged towards one another into the tissue clamping configuration to secure the stimulation device 10 to tissue supported between the inner and outer electrodes 22, 32. Specifically, a force is created by the biasing member 42 of the pivot 40 that urges the electrodes 22, 32 towards each other to clamp or clasp tissue between the electrodes 22, 32, and also secure the stimulation device 10 on the ear of a patient. The electrodes 22, 32 are positioned to overlay outer ear tissue (e.g., the auricle) innervated by the auricular branch of the vagus nerve when the stimulation device 10 is clamped to the ear.

With continued reference to FIG. 3, the outer electrode 32 of the outer probe 30 is offset from the inner electrode 22 of the inner probe 20. Offsetting the inner and outer electrodes 22, 32 provides improved stimulation of the auricular branch of the vagus nerve when both electrodes 22, 32 are activated and when one of the two electrodes 22, 32 is activated, as compared to having the electrodes 22, 32 directly oppose one another.

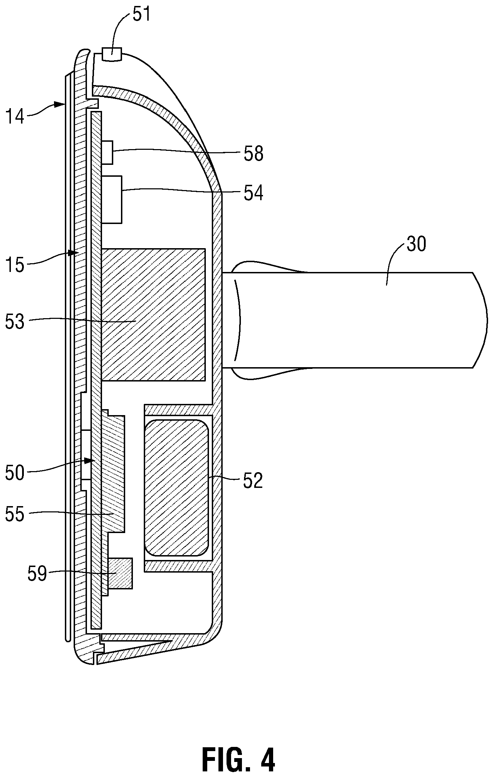

With continued reference to FIGS. 2-4, one or both of the inner and outer probes 20, 30 and/or the housing 12 of the stimulation device 10 may house a circuit board 50 of the stimulation device 10 with associated electronics for coordinating the activation of the electrodes 22, 32 of the inner and outer probes 20, 30, either separately or together. The circuit board 50 may have several elements including, but not limited to, a rechargeable power source 52, a stimulation circuit 54, electrode outputs 56 (FIG. 2) in operative communication with the stimulation circuit 54, a control interface 58, and a memory 59. The circuit board 50 may also include a power switch 51 and a corresponding status indicator 49 (FIG. 1) that extends from the housing 12 for being accessible by a patient or clinician. The circuit board 50 may also include an over-current protection circuit (not shown) and/or an over-heating protection circuit (not shown). The status indicator 49 of the circuit board 50 indicates whether the power switch 51 is in the "on" or "off position. For example, the status indicator 49 can be an LED that is illuminated when the power switch 51 is in the "on" position and not illuminated when the power switch 51 is in the 'off" position.

The circuit board 50 of the stimulation device 10 also includes a controller 55, such as a microcontroller, for controlling the operation of the stimulation device 10. The controller 55 can be programmed with a variety of operating protocols and parameters. The operating protocols and parameters can be stored in the memory 59 or in a memory of controller 55. The memory 59 and/or the controller's memory store data, such as data logged during usage of the stimulation device 10 including, but not limited to, duration of use, time of day of use, and operating parameters of the stimulation circuit 54.

One or more elements of the circuit board 50 of the stimulation device 10 may be integrated into an application-specific integrated circuit (ASIC) or controller 55. By integrating and packaging elements of the circuit board 50 into and ASIC or controller 55, the wiring and/or size of one or both of the inner and outer probes 20, 30 may be reduced. In addition, the integration of elements into an ASIC or controller 55 may increase the battery life of the stimulation device 10. Alternatively, elements of the circuit board 50 and the controller 55 can be housed in a separate unit other than the stimulation device 10, and wirelessly or by wired connection communicate with the stimulation circuit 54 of the stimulation device 10. For example, the stimulation circuit 54 can be powered and controlled inductively via inductive coupling by control circuitry positioned in proximity to the stimulation device 10.

The rechargeable power source 52 of the circuit board 50 may be a battery or other device suitable for providing power to elements of the circuit board 50 and/or controller 55. The rechargeable power source 52 may be recharged through direct contact with an external power source (e.g., via a selectively removable power chord that may couple to a port such as a USB (not shown) supported by housing 12) or may be inductively charged by positioning a suitable power source adjacent the respective inner or outer probe 20, 30. The rechargeable power source 52 may be in communication with an induction coil 53 that receives power by inductive coupling from an external power supply (not shown) for recharging the power source 52. In some embodiments, the rechargeable power source 52 may include one or more photovoltaics to enable recharging by light energy.

The stimulation circuit 54 of the circuit board 50 is configured to generate stimulation signals, such as pulses, oscillations, sinusoidal waveforms, square waveforms, triangular waveforms, etc., when activated, and to transmit the stimulation signals via the electrode outputs 56 to one or both of the electrodes 22, 32 of the inner and outer probes 20, 30, respectively. The stimulation circuit 54 can be an oscillator or other electronic element configured to produce a signal or pulses which can stimulate the auricular branch of the vagus nerve to treat a predetermined condition. In some embodiments, the circuit board 50 may have a circuit configured to sense when the electrodes 22, 32 are clamped to tissue, such as by an impedance measurement, and to prevent actuation of the stimulation circuit 54 when tissue is not clamped between the electrodes 22, 32.

Each of the electrode outputs 56 of the circuit board 50 connected to the stimulation circuit 54 is in communication with a respective one of the electrodes 22, 32 of the inner and outer probes 20, 30 to deliver a stimulation signal, such as, for example, a waveform or a series of pulse bursts, generated by the stimulation circuit 54 to the electrodes 22, 32. The stimulation signal causes one or both of the electrodes 22, 32 to transcutaneously stimulate the auricular branch of the vagus nerve within the tissue. In certain embodiments, the electrodes 22, 32 may sense when they are in contact with tissue, for instance, an ear of a patient, and provide a tissue sensing signal via the electrode outputs 56 to the stimulation circuit 54 or the controller 50. If the stimulation circuit 54 or the controller 55 does not receive the tissue sensing signal from one or both electrodes 22, 32, the stimulation circuit 54 will not be actuated.

Besides the controller 55 of the circuit board 50, the stimulation circuit 54 of the circuit board 50 can also be controlled by an external device via the control interface 58 as detailed below. The controller 55 or external device can enable fine control of the operating parameters of the stimulation circuit 54, including, but not limited to, the duration, the amplitude, the frequency, the type of stimulation or oscillation waveform, or burst rate of the pulses, etc. Additionally or alternatively, the stimulation device 10 may be provided without the controller 55 such that one or more elements of the circuit board 50 are controlled by an external controller or an external control mechanism, such as by the controller 122 (FIG. 5) of the smart device 120.

The stimulation circuit 54 and other elements of the circuit board 50, including the controller 55, can be controlled or programmed using an app running on a smart device 120 (FIG. 5) or other electronic device (e.g., external controller). The app through the control interface 58, for example, can receive and transmit control signals wirelessly (e.g., WIFI signals) and program and/or control the stimulation circuit 54 and/or the controller 55 in order for the stimulation device 10 to generate a stimulation signal for treating the predetermined condition by stimulating the auricular branch of the vagus nerve. The stimulation device 10 can be connected via the control interface 58 or other communication circuitry to the internet or other network for receiving the control signals. In this case, the stimulation device 10 can be an internet of things (IoT) device configured to be remotely controlled via a network connection.

The control interface 58 of the circuit board 50 may be a wireless transmitter/receiver in wireless communication with the smart device 120 or external controller. The wireless communication may be radio frequency, optical, WIFI, BLUETOOTH® (an open wireless protocol for exchanging data over short distances (using short length radio waves) from fixed and mobile devices, ZigBee® (a specification for a suite of high level communication protocols using small, low-power digital radios based on the IEEE 802.15.4-2003 standard for wireless personal area networks (WPANs)), etc.

As stated above, the control interface 58 of the circuit board 50 may link to a smart device 120. Through the link, the control interface 58 may transfer data from the memory 59 and/or real-time data from the stimulation circuit 54 to the smart device 120. The controller 55 of the circuit board 50 may also receive control signals from the smart device 120 via the communication link for controlling the stimulation circuit 54 of the circuit board 50. The smart device 120 through the app may visually or audibly present data from the stimulation device10 to a clinician in real-time or other individual, including the patient. For example, a GUI of the app can provide visual information, such as the type of condition being treated and the operating parameters of the stimulation circuit 54 of the circuit board 50, such as the frequency and amplitude of the stimulation signal generated by the stimulation circuit 54.

With reference to FIG. 5, a system 100 is provided which can include the features described herein above and other features, and configured for stimulating the auricular branch of the vagus nerve and treating a condition in accordance with the present disclosure. The system 100 includes an auricular stimulation device 10 (see FIGS. 1A and 1B), a monitoring device 110, and a smart device 120. The auricular stimulation device 10 can be the device described above with reference to FIGS. IA-4, or it can be any stimulation device for stimulating the auricular branch of the vagus nerve, e.g., auricular stimulation. The monitoring device 110 is any monitoring device suitable for measuring, monitoring, and/or determining biomarker information of a patient, such as heart rate, respiration rate, electro-dermal activity, neural activity, EEG, EKG, glucose level, cholesterol, blood pressure, levels of cytokines present, and/or other physiological measurements or molecular or enzyme-related information corresponding to the patient. For example, the monitoring device 110 may be an implanted sensor within the patient for measuring the patient's blood glucose level. The monitoring device 110 may also be a wearable device, such as a smart watch, or a finger pulse oximeter which monitors and determines the heart rate and respiration rate of a patient.

The smart device 120 of the system 100 may be, but not limited to, a smartphone, a portable computer, a tablet, a fixed computer, or a wearable device connected to a network, such as the internet, and/or operating under a communications protocol, such as BLUETOOTH™. In some embodiment, the smart device 120 may be an external controller configured to communicate wirelessly or via a wired connection and control the stimulation device 10.

With continued reference to FIG. 5, the communications links between the smart device 120 and the stimulation device 10 and monitoring device 110 of the system 100 can be wireless or non-wireless. A processor 124 of the smart device 120 receives biomarker information from the monitoring device 110. The biomarker information can be used by the smart device 120 to determine the type of stimulation signal configured to treat the patient's condition. The processor 124 then communicates with a controller 122 of the smart device 122 to transmit control signals to the stimulation device 10, either wirelessly or non-wirelessly. The control signals are received by the controller interface 58 and are used to control the stimulation circuit 54 for generating the processor-determined stimulation signal.

As stated above, the smart device 120 of the system 100 may also receive data from the memory 59 of the stimulation device 10, such as previous usage data, operating parameters of the stimulation device 10 during a previous treatment session or cycle, etc. which can aid the processor 124 to determine the most effective stimulation signal and associated operating parameters of the stimulation circuit 54 for treating the patient. Alternatively or additionally, user input 126 can be received by the smart device 120 via a GUI of an app to operate the stimulation circuit 54 based on user-selected operating parameters.

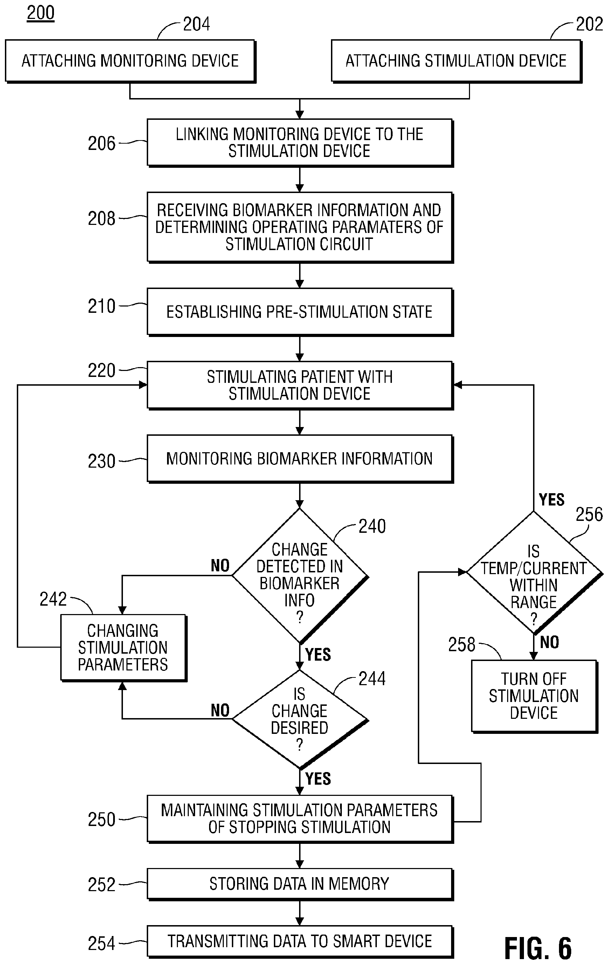

With reference to FIG. 6, an exemplary method 200 of treating a condition is described in accordance with the present disclosure with reference to the system 100 detailed above. Other methods of treatment are contemplated and envisioned using the stimulation device 10 and system 100 described herein. For example, as shown the stimulation device 10 is configured for attachment to the left ear of a patient; however, the stimulation device 10 may be configured as the "mirror image" of the device shown, and attached to the right ear of the patient. Alternatively, two stimulation devices 10 may be configured as "mirror images" of each other, and each attached to a respective ear of a patient and simultaneously or sequentially used to stimulate the auricular vagus innervation bilaterally, as detailed below.

Initially, with continued reference to FIG. 6, the stimulation device 10 and the monitoring device 110 of the system 100 are attached to the patient such that the stimulation device 10 is positioned to stimulate the vagus nerve, for example, the auricular branch of the vagus nerve in the ear of the patient (step 202), and the monitoring device 110 is positioned to monitor and determine biomarker information of the patient, for example, the heart rate and/or the respiration rate of the patient (step 204). Where the monitoring device 110 is an implantable device, such as a glucose monitoring implantable device, the monitoring device 110 includes transmission circuitry for transmitting the biomarker information, such as blood glucose level to the processor 124 of the smart device 120.

With the stimulation device 10 and the monitoring device 110 in position, the smart device 120 can be linked to the stimulation device 10 and the monitoring device 110 (step 206). The smart device 120 can be in wireless communication with the stimulation device 10 and the monitoring device 110; however, in embodiments, the smart device 120 may be physically linked or hardwired to the stimulation device 10 and the monitoring device 110. In certain embodiments, the smart device 120 is linked to the stimulation device 10 and the monitoring device 110 prior to the stimulation device 10 and the monitoring device 110 being in position.

When the stimulation device 10 of the system 100 is positioned about the ear, the stimulation device 10 may sense tissue properties via the inner and/or outer electrode 22, 32 of the inner and outer probes 20, 30 and internally calibrate one or more operating parameters in response to the sensed tissue properties. The operating parameters can also be calibrated or initially determined using the initial biomarker information received from the monitoring device 110, and/or user input 126 (step 208). The initial biomarker information refers to information received prior to stimulating the auricular branch of the vagus nerve. The initial biomarker information received by the smart device 120 from the monitoring device 110 can be used to establish a pre-stimulated state of the patient (step 210). That is, the state of the patient prior to stimulation of the auricular branch of the vagus nerve. This state corresponds to the patient having a condition which necessitates the patient be treated by stimulating the auricular branch of the vagus nerve. Therefore, it is the objective of the treatment method to stimulate the vagus nerve to treat the patient and adjust the patient's pre-stimulated state to a post-stimulated state which is healthier than the pre-stimulated state.

Based on at least the initial biomarker information, the smart device 120 of the system 100 can access one or more databases or a memory which correlates the initial biomarker information to a plurality of conditions, and determine one or more conditions of the patient, such as high blood pressure, high blood glucose level, high temperature, etc., based on the initial biomarker information. Therefore, the smart device 120 is configured to diagnose or determine one or more conditions of the patient using the initial biomarker information.

Once one or more conditions are determined by the smart device 120, the smart device can access one or more additional databases or the same databases, or a memory, which correlate a plurality of conditions with a plurality of treatment regimens or protocols. All of the databases referred to herein can be accessed by the smart device 120 or other computing device via a network connection, such as the internet.

For example, the smart device 120 of the system 100 can access a variety of treatment regimens stored within one or more databases stored in a remote location (i.e., cloud-based network architecture). The databases can be stand-alone databases or data structures stored in a remote server or other computing device. After accessing the plurality of treatment regimens stored within the one or more databases, the smart device 120 or other computing device selects the treatment regimen that is most suitable for treating a patient having the determined condition(s) of the patient. For example, if the initial biomarker information indicates the patient has a high glucose level, the smart device 120 or other computing device selects the treatment regimen which has been previously determined to be effective in treating patients with a high glucose level. The treatment regimen selected can be tailored or adjusted based on other information gleaned from the patient's biomarker information or other information related to the patient, including, but not limited to, blood pressure and heart rate of the patient, musculoskeletal stability, age of the patient, medical history, prescription medication(s) administered to the patient, etc. The treatment regimen can also be manually selected or tailored by a clinician, and communicated to the smart device 120 via the user input 126 (e.g., via a graphical user interface) or other controller in operative communication with the stimulation device 10.

Each treatment regimen can include, but not limited to, the operating parameters of the stimulation circuit 54, such as, the type of waveform and corresponding characteristics (e.g., frequency and amplitude), the duration of the treatment session, and the number of treatment sessions.

After the treatment regimen is selected or determined, and/or adjusted or tailored to the patient being treated, either by a clinician, the smart device 120 or other computing device, the controller 122 of the smart device 120 or other controller in operative communication with the stimulation device 10 transmits a control signal to the controller interface 58 of the stimulation device 10 to begin the treatment session and treat the patient in accordance with the treatment regimen (step 220).

The control signal may include parameters for the desired stimulation in accordance with the treatment regimen. In response to receiving the control signal via the controller interface 58, the controller 55 controls the stimulation circuit 54 to generate and deliver a waveform, a series of pulses or other stimulating signals to the inner and/or outer electrodes 22, 32 via the electrode outputs 56 to transcutaneously stimulate the vagus nerve, i.e., the auricular branch of the vagus nerve.

As the stimulation device 10 non-invasively stimulates the vagus nerve, the smart device 120 receives and monitors biomarker information, e.g., heart rate and/or respiration rate of the patient, via the monitoring device 110, continuously in real-time or at pre-set intervals (step 230). In some embodiments, the controller interface 58 of the stimulation device 10 can also receive the biomarker information from the monitoring device 10.

In response to the stimulation, the smart device 120 may detect a change (or detect no change, or detect no significant therapeutic change) in the biomarker information received from the monitoring device 110 (step 240). If no change or no significant therapeutic change is determined in the biomarker information, the smart device 120 sends a control signal to the auricular stimulation device 10 to change or adjust the stimulation parameters (step 242). The method then proceeds to step 220 where the patient is stimulated with the stimulation device 10 using the new stimulation parameters.

For example, when no change or no significant therapeutic change is detected in the biomarker information in step 240, the smart device 120 may increase the duration, amplitude, frequency, and/or burst rate of the pulses or other signal parameters, change the type of waveform, etc. until a desired or noticeable therapeutic change is detected in the biomarker information (step 244). These stimulation parameters are then maintained (step 250) and stimulation continues in step 220.

However, before continuing with stimulation using the new stimulation parameters in step 220, the system may check to determine if the temperature and current of the stimulation device 10 are within acceptable ranges (step 256). The stimulation device 10 is turned off if the temperature and/or current are outside acceptable ranges (step 258).

If in step 244, a change is detected in the biomarker information of the patient and the biomarker information is within an acceptable range, for instance, heart rate and/or the respiration rate of the patient is in the normal range, the smart device 120 may control the stimulation device 10 to cease the operation of the stimulation circuit 54 (step 250) and conclude the treatment session. That is, if it is determined by the smart device 120 that the stimulation treatment was effective in bringing the initial biomarker information of the pre-stimulated state of the patient within an acceptable range, the stimulation treatment session is finished.

The data acquired during the treatment session, including the stimulation parameters which were effective in treating the patient's condition can be stored in memory 59, smart device 120 or other computing device (steps 252, 254). The stimulation device 10 or smart device 120 may also be programmed to operate the stimulation circuit 54 in a future treatment session using the stimulation parameters that brought the initial biomarker information within the normal or accepted range.

The stimulation parameters can also be transmitted to a remote server and stored in a data structure or in the one or more databases for being accessed by clinicians as a set of treatment parameters for a given condition. Hence, over time, a "smart" database or artificial intelligence (AI) system is built having a plurality of sets of treatment parameters corresponding to the treatment of a plurality of conditions and patient characteristics (e.g., age, musculoskeletal stability, etc.); that is, the database or AI system can eliminate or shorten the treatment sessions for many patients since the most optimum treatment and stimulation parameters for a plurality of conditions will be known in advance.

In some embodiments, the smart device 120 of the system 100 may provide visual and/or audible feedback to the patient and/or a clinician before, during, and/or after a treatment session.

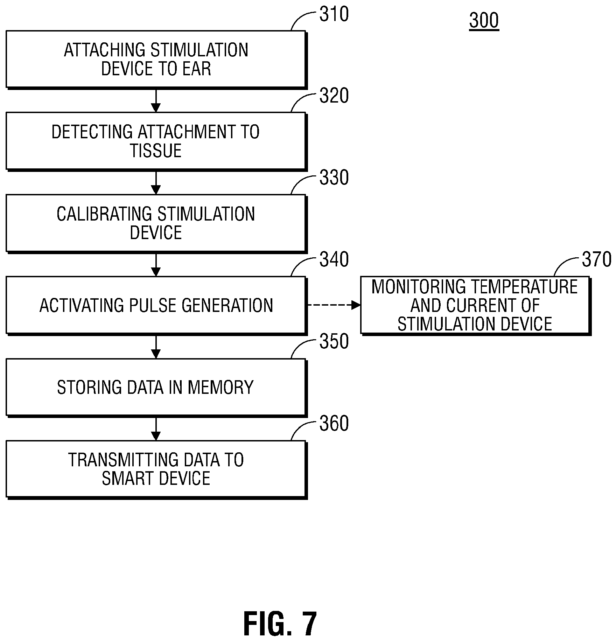

With reference to FIG. 7, an exemplary treatment method 300 of stimulating a vagus nerve is disclosed using pulses as the stimulation signal in accordance with the present disclosure and with reference to the stimulation device 10 of FIGS. 1A-4. Initially, the stimulation device 10 is attached to the ear of a patient such that the inner and outer electrodes 22, 32 of the inner and outer probes 20, 30, respectively, are positioned in opposition and offset from one another (step 310). The patient or a third party, such as a clinician, may position the stimulation device 10 on the ear of a patient. When the stimulation device 10 is positioned on the ear of the patient, the inner and outer electrodes 22, 32 are positioned about the auricular branch of the vagus nerve.

With the stimulation device 10 positioned and the power switch 51 has been set to the "on" position to allow operation of the stimulation device 10, the stimulation circuit 54 is activated to deliver pulses to the electrode outputs 56 which are transmitted from the inner and/or outer electrode 22, 32 of the inner and outer probes 20, 30, respectively. The stimulation circuit 54 may detect when the stimulation device 10 is attached to the tissue, e.g., an ear, and self-activate or by control of the smart device 120 to deliver therapeutic pulses to the auricular branch of the vagus nerve (step 320). For example, a circuit may be completed between the inner and outer electrodes 22, 32 as tissue is clamped therebetween.

The offset between the inner and outer electrodes 22, 32 of the inner and outer probes 20, 30, respectively, may prevent the circuit from being completed when the stimulation device 10 is in a clamped configuration with no tissue disposed between the inner and outer electrodes 22, 32. After interposed tissue is detected and before the therapeutic pulses are delivered, the stimulation circuit 54 may calibrate to tissue between the inner and outer electrodes 22, 32 by sending calibrating pulses from one of the inner or outer electrodes 22, 32 to the other and determining a resistance of tissue between the inner and outer electrodes 22, 32 (step 330). The resistance between the inner and outer electrodes 22, 32 may be used to determine one of the parameters of the therapeutic pulses to be delivered.

After activation of the stimulation circuit 54, the stimulation circuit 54 generates and delivers pulses to the inner and/or outer electrodes 22, 32 for a predetermined amount of time or until tissue is not detected between the two electrodes (e.g., the user removed the stimulation device 10 from the ear) (step 340). As the stimulation circuit 54 delivers pulses to the inner and/or outer electrodes 22, 32, the memory 58 may record data of the delivered pulses including, but not limited to, calibration data, time tissue detected, duration of tissue detection, and parameters of the pulses delivered (step 350).

As the stimulation circuit 54 delivers pulses, the stimulation device 10, e.g., the controller 50 may conduct safety checks (step 370). For example, the controller 50 or circuitry may verify whether the current being drawn from the power source 52 is below a maximum allowed current level. If the current being drawn from the power source 52 exceeds the maximum allowed current, the controller 50 may cease the operation of the stimulation circuit 54. Additionally or alternatively, the controller 50 or circuitry may monitor the temperature of the stimulation device 10, such that if the temperature of the stimulation device 10 exceeds a maximum allowed temperature, the controller 50 may cease operation of the stimulation circuit 54. This treatment method may be repeated multiple times over an extended period of time allowing for out-patient procedures to be completed between visits to a clinician's office.

During or after the delivery of pulses to the auricular branch of the vagus nerve by the stimulation device 10, the stimulation device 10 may link with a smart device 120 which receives data from the memory 59 including information related to one or more treatment sessions, such as, for example, operating parameters of the stimulation circuit 54 (step 360). The smart device 120 may be the patient's smart device and transmit the data to a clinician. Additionally or alternatively, the smart device 120 may be a clinician's such that at periodic visits, the clinician links to the stimulation device 10 to receive data from the memory 59 of the stimulation device 10. The clinician may analyze the data and update or change one or more operating parameters of the stimulation circuit 54 to update or change one or more characteristics of the pulses generated and delivered by the stimulation circuit 54. The clinician may use the smart device 120, e.g., in the clinician's office and/or remotely via a network connection, to connect with the stimulation device 10 and update or change the operating parameters of the stimulation circuit 54 to update or change one or more characteristics of the pulses.

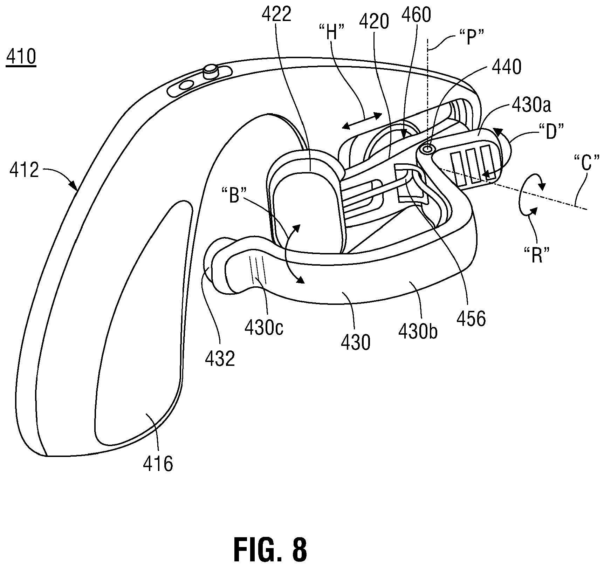

Turning now to FIG. 8, another embodiment of a StimClip™ or auricular stimulation device 410 is illustrated that is substantially similar to stimulation device 10 and is only described herein to the extent necessary to describe the differences in operation and construction of stimulation device 410. In general, stimulation device 410 includes a housing 412, a first or inner arm 420 (e.g. probe), a second or outer arm 430 (e.g., probe), and an adjustment mechanism 460 that couples inner and outer probes 420, 430 to housing 412 and enables horizontal and rotational adjustment of probes 420, 430 relative to housing 412, as indicated by arrows "H" and "R," respectively. The inner and outer probes 420, 430 are pivotally coupled together about pivot axis "P" to selectively clamp electrodes 422, 432 of respective inner and outer probes 420, 430 to an ear while the housing 412 is supported on the ear. More specifically, the outer probe 430 pivots relative to first probe 420 about pivot axis "P" to move probes 420, 430 between a clamped position and an unclamped position, as indicated by arrows "B."

The outer probe 430 of the stimulation device 410 includes a foot 430a that facilitates pivotal movement of outer probe 430 about pivot axis "P," as detailed above with respect to outer probe 30 of the stimulation device 410. The outer probe 430 further includes an arched or pre-bent body 430b and a head 430c that supports the electrode 432 of the outer probe 430. The foot 430a, pre-bent body 430b, and the head 430c of the outer probe 430 are formed of rigid material to prevent flexing of outer probe 430.

As can be appreciated, securement of any of the components of the presently disclosed apparatus can be effectuated using known securement techniques such welding, crimping, adhesion, fastening, etc.

### APPENDIX

- George, R., Sonnen, A., Upton, A., Salinsky, M., Ristanovic, R., Bergen, D., Mirza, W., Rosenfeld, W., Nari-Toku, D., Manon-Espaillat, R. and Barolat, G., 1995, "A randomized controlled trial of chronic vagus nerve stimulation for treatment of medically intractable seizures," Neurology, 45(2), pp.224-230.
- Morris, G.L. and Mueller, W.M., 1999, "Long-term treatment with vagus nerve stimulation in patients with refractory epilepsy," Neurology, 53(8), pp.1731-1731.
- Labar, D., Murphy, J., Tecoma, E. and E VNS Study Group, 1999, "Vagus nerve stimulation for medication-resistant generalized epilepsy," Neurology, 52(7), pp.1510-1510.
- Sackeim, H.A., Rush, A.J., George, M.S., Marangell, L.B., Husain, M.M., Nahas, Z., Johnson, C.R., Seidman, S., Giller, C., Haines, S. and Simpson, R.K., 2001, "Vagus nerve stimulation (VNS™) for treatment-resistant depression: efficacy, side effects, and predictors of outcome," Neuropsychopharmacology, 25(5), pp.713-728.
- Nemeroff, C.B., Mayberg, H.S., Krahl, S.E., McNamara, J., Frazer, A., Henry, T.R., George, M.S., Charney, D.S. and Brannan, S.K., 2006, "VNS therapy in treatment-resistant depression: clinical evidence and putative neurobiological mechanisms," Neuropsychopharmacology, 31(7), p.1345.
- Nahas, Z., Marangell, L.B., Husain, M.M., Rush, A.J., Sackeim, H.A., Lisanby, S.H., Martinez, J.M. and George, M.S., 2005, "Two-year outcome of vagus nerve stimulation (VNS) for treatment of major depressive episodes," The Journal of clinical psychiatry, 66(9), pp. 1097-1104.
- De Ridder, D., Vanneste, S., Engineer, N.D. and Kilgard, M.P., 2014, "Safety and efficacy of vagus nerve stimulation paired with tones for the treatment of tinnitus: a case series," Neuromodulation: Technology at the Neural Interface, 17(2), pp.170-179.
- Stavrakis S, Humphrey MB, Scherlag BJ, Hu Y, Jackman WM, Nakagawa H, Lockwood D, Lazzara R, Po SS (Mar 2015), "Low-level transcutaneous electrical vagus nerve stimulation suppresses atrial fibrillation," J Am Coll Cardiol. 65: 867-75.
- Hein E, Nowak M, Kiess O, Biermann T, Bayerlein K, Kornhuber J, Kraus T (May 2013), "Auricular transcutaneous electrical nerve stimulation in depressed patients: a randomized controlled pilot study," J Neural Transm (Vienna). 120: 821-7.
- Huang F, Dong J, Kong J, Wang H, Meng H, Spaeth RB, Camhi S, Liao X, Li X, Zhai X, Li S, Zhu B, Rong P (June 2014), "Effect of transcutaneous auricular vagus nerve stimulation on impaired glucose tolerance: a pilot randomized study," BMC Complement Altern Med. 14: 203.
- Kreuzer PM, Landgrebe M, Resch M, Husser O, Schecklmann M, Geisreiter F, Poeppl TB, Prasser SJ, Hajak G, Rupprecht R, Langguth B (September 2014), "Feasibility, safety and efficacy of transcutaneous vagus nerve stimulation in chronic tinnitus: an open pilot study," Brain Stimul. 7: 740-7.

## Claims

1. An auricular stimulation device (10) comprising:
a first electrode (22) disposed on a first arm (20);
a second electrode (32) disposed on a second arm (30), wherein the second electrode is in opposition to the first electrode and offset from the first electrode;
a biasing member (42) configured to urge a portion of the first arm towards a portion of the second arm, wherein at least one of the first and second electrodes is urged towards the other electrode to form a tissue clamping configuration; and
a stimulation circuit (54) in operative communication with the first and second electrodes, wherein the stimulation circuit is configured for generating a stimulation signal for actuating at least one of the first and second electrodes for stimulating a nerve within tissue clamped between the first and second electrodes; and
an over-current protection circuit in operative communication with the stimulation circuit, wherein the over-current protection circuit is configured to cease operation of the stimulation circuit in an over-current situation.

2. The stimulation device according to claim 1, further comprising a controller in operative communication with the stimulation circuit for controlling operation of the stimulation circuit.

3. The stimulation device according to claim 2, further comprising a housing, wherein the controller and the stimulation circuit are disposed within the housing.

4. The stimulation device according to claim 3, wherein the housing is configured to be positioned about an ear of a patient.

5. The stimulation device according to claim 1, further comprising an adjustment mechanism partially disposed within the housing and including the biasing member, wherein the adjustment mechanism enables the position of at least one of the arms to be changed for enabling the stimulation device to fit and conform to a variety of ears.

6. The stimulation device according to claim 1, further comprising a control interface for receiving at least one control signal from an external device for controlling the stimulation circuit.

7. The stimulation device according to claim 6, wherein the external device is a smart device.

8. The stimulation device according to claim 1, further comprising a power source in operative communicative with the stimulation circuit.

9. The stimulation device according to claim 2, further comprising a memory in operative communication with the controller, wherein the memory is configured for storing usage data and operating parameters of the stimulation device.

10. A diagnostic and therapeutic system (100) comprising:
a smart device (120); and
a stimulation device (10) according to claim 1, in operative communication with the smart device and configured to receive at least one control signal from the smart device,
wherein the stimulation circuit (54) is configured for generating a stimulation signal after receiving the at least one control signal for actuating at least one of the first and second electrodes for stimulating a nerve within tissue clamped between the first and second.

11. The system according to claim 10, wherein the smart device is in operative communication with at least one of a memory and a database storing a plurality of treatment regimens corresponding to a plurality of conditions.

12. The system according to claim 10, further comprising a monitoring device in operative communication with at least one of the smart device and the stimulation device, wherein the monitoring device transmits biomarker information to the at least one of the smart device and the stimulation device.

13. The system according to claim 12, wherein the smart device determines at least one condition of a patient using the biomarker information, and wherein the smart device determines at least one treatment regimen from the plurality of treatment regimens for treating the at least one condition of the patient.

14. The system according to claim 10, wherein the smart device includes at least one app having a corresponding graphical user interface for receiving at least one user input for controlling at least one operating parameter of the stimulation device.

15. The stimulation device of claim 1, wherein at least one of the first and second arms is configured to move between at least one bent configuration and an unbent configuration.

## Patentansprüche

1. Aurikuläre Stimulationsvorrichtung (10), umfassend:
eine erste Elektrode (22), die an einem ersten Arm (20) angeordnet ist;
eine zweite Elektrode (32), die an einem zweiten Arm (30) angeordnet ist, wobei die zweite Elektrode der ersten Elektrode gegenüberliegt und von der ersten Elektrode versetzt ist;
ein Vorspannelement (42), das so konfiguriert ist, dass es einen Abschnitt des ersten Arms in Richtung eines Abschnitts des zweiten Arms drängt, wobei mindestens eine der ersten und zweiten Elektroden in Richtung der anderen Elektrode gedrängt wird, um eine Gewebeklemmkonfiguration zu bilden; und
eine Stimulationsschaltung (54), die in operativer Kommunikation mit der ersten und der zweiten Elektrode steht,
wobei die Stimulationsschaltung so konfiguriert ist, dass sie ein Stimulationssignal zum Betätigen mindestens einer der ersten und zweiten Elektroden erzeugt, um einen Nerv innerhalb des zwischen der ersten und zweiten Elektrode eingeklemmten Gewebes zu stimulieren; und
eine Überstromschutzschaltung in operativer Kommunikation mit der Stimulationsschaltung,
wobei die Überstromschutzschaltung so konfiguriert ist, dass sie den Betrieb der Stimulationsschaltung in einer Überstromsituation unterbricht.

2. Stimulationsvorrichtung nach Anspruch 1, ferner umfassend eine Steuerung in operativer Kommunikation mit der Stimulationsschaltung zur Steuerung des Betriebs der Stimulationsschaltung.

3. Stimulationsvorrichtung nach Anspruch 2, ferner umfassend ein Gehäuse, wobei die Steuereinheit und der Stimulationsschaltkreis innerhalb des Gehäuses angeordnet sind.

4. Stimulationsvorrichtung nach Anspruch 3, wobei das Gehäuse so konfiguriert ist, dass es um ein Ohr eines Patienten positioniert werden kann.

5. Stimulationsvorrichtung nach Anspruch 1, ferner umfassend einen Einstellmechanismus, der teilweise innerhalb des Gehäuses angeordnet ist und das Vorspannelement enthält, wobei der Einstellmechanismus es ermöglicht, die Position von mindestens einem der Arme zu verändern, um die Stimulationsvorrichtung in die Lage zu versetzen, sich an eine Vielzahl von Ohren anzupassen und zu passen.

6. Stimulationsvorrichtung nach Anspruch 1, ferner umfassend eine Steuerschnittstelle zum Empfangen mindestens eines Steuersignals von einer externen Vorrichtung zum Steuern der Stimulationsschaltung.

7. Die Stimulationsvorrichtung nach Anspruch 6, wobei die externe Vorrichtung eine intelligente Vorrichtung ist.

8. Stimulationsvorrichtung nach Anspruch 1, ferner umfassend eine Energiequelle in operativer Kommunikation mit dem Stimulationsschaltkreis.

9. Stimulationsvorrichtung nach Anspruch 2, ferner umfassend einen Speicher in operativer Kommunikation mit der Steuereinheit, wobei der Speicher zum Speichern von Nutzungsdaten und Betriebsparametern der Stimulationsvorrichtung konfiguriert ist.

10. Diagnostisches und therapeutisches System (100), umfassend:
eine intelligente Vorrichtung (120); und
eine Stimulationsvorrichtung (10) nach Anspruch 1 in operativer Kommunikation mit der intelligenten Vorrichtung und so konfiguriert, dass sie mindestens ein Steuersignal von der intelligenten Vorrichtung empfängt,
wobei die Stimulationsschaltung (54) so konfiguriert ist, dass sie nach dem Empfang des mindestens einen Steuersignals ein Stimulationssignal erzeugt, um mindestens eine der ersten und zweiten Elektroden zu betätigen, um einen Nerv innerhalb des zwischen der ersten und zweiten Elektrode eingeklemmten Gewebes zu stimulieren.

11. System nach Anspruch 10, wobei die intelligente Vorrichtung in operativer Kommunikation mit mindestens einem von einem Speicher und einer Datenbank, die eine Vielzahl von Behandlungsschemata speichern, die einer Vielzahl von Zuständen entsprechen.

12. System nach Anspruch 10, das ferner eine Überwachungsvorrichtung umfasst, die in operativer Kommunikation mit mindestens einer von der intelligenten Vorrichtung und der Stimulationsvorrichtung steht, wobei die Überwachungsvorrichtung Biomarkerinformationen an die mindestens eine von der intelligenten Vorrichtung und der Stimulationsvorrichtung überträgt.

13. System nach Anspruch 12, wobei die intelligente Vorrichtung mindestens einen Zustand eines Patienten unter Verwendung der Biomarkerinformationen bestimmt, und wobei die intelligente Vorrichtung mindestens ein Behandlungsschema aus der Vielzahl von Behandlungsschemata zur Behandlung des mindestens einen Zustands des Patienten bestimmt.

14. System nach Anspruch 10, wobei die intelligente Vorrichtung mindestens eine App mit einer entsprechenden grafischen Benutzeroberfläche zum Empfangen mindestens einer Benutzereingabe zum Steuern mindestens eines Betriebsparameters der Stimulationsvorrichtung enthält.

15. Stimulationsvorrichtung nach Anspruch 1, wobei mindestens einer der ersten und zweiten Arme so konfiguriert ist, dass er sich zwischen mindestens einer gebogenen Konfiguration und einer nicht gebogenen Konfiguration bewegt.

## Revendications

1. Dispositif de stimulation auriculaire (10) comprenant :
une première électrode (22) disposée sur un premier bras (20) ;
une seconde électrode (32) disposée sur un second bras (30), dans lequel la seconde électrode est en opposition à la première électrode et décalée par rapport à la première électrode ;
un élément de prétension (42) configuré pour pousser une partie du premier bras vers une partie du second bras, dans lequel au moins une des première et seconde électrodes est poussée vers l'autre électrode pour former une configuration de pincer de tissu ; et
un circuit de stimulation (54) en communication opérationnelle avec les première et deuxième électrodes,
dans lequel le circuit de stimulation est configuré pour générer un signal de stimulation pour actionner au moins une des première et seconde électrodes pour stimuler un nerf à l'intérieur du tissu pincé entre les première et seconde électrodes ; et
un circuit de protection contre les surintensités en communication opérationnelle avec le circuit de stimulation,
dans lequel le circuit de protection contre les surintensités est configuré pour cesser le fonctionnement du circuit de stimulation dans une situation de surintensité.

2. Dispositif de stimulation selon la revendication 1, comprenant en outre un contrôleur en communication opérationnelle avec le circuit de stimulation pour commander le fonctionnement du circuit de stimulation.

3. Dispositif de stimulation selon la revendication 2, comprenant en outre un boîtier, dans lequel le contrôleur et le circuit de stimulation sont disposés à l'intérieur du boîtier.

4. Dispositif de stimulation selon la revendication 3, dans lequel le boîtier est configuré pour être positionné autour d'une oreille d'un patient.

5. Dispositif de stimulation selon la revendication 1, comprenant en outre un mécanisme de réglage partiellement disposé à l'intérieur du boîtier et comprenant l'élément de prétension, dans lequel le mécanisme de réglage permet de modifier la position d'au moins un des bras pour permettre au dispositif de stimulation de s'adapter et de se conformer à une variété d'oreilles.

6. Dispositif de stimulation selon la revendication 1, comprenant en outre une interface de commande pour recevoir au moins un signal de commande provenant d'un dispositif externe pour commander le circuit de stimulation.

7. Dispositif de stimulation selon la revendication 6, dans lequel le dispositif externe est un dispositif intelligent.

8. Dispositif de stimulation selon la revendication 1, comprenant en outre une source d'énergie en communication opérationnelle avec le circuit de stimulation.

9. Dispositif de stimulation selon la revendication 2, comprenant en outre une mémoire en communication opérationnelle avec le contrôleur, dans lequel la mémoire est configurée pour stocker des données d'utilisation et des paramètres de fonctionnement du dispositif de stimulation.

10. Système de diagnostic et thérapeutique (100) comprenant :
un dispositif intelligent (120) ; et
un dispositif de stimulation (10) selon la revendication 1, en communication opérationnelle avec le dispositif intelligent et configuré pour recevoir au moins un signal de commande du dispositif intelligent,
dans lequel le circuit de stimulation (54) est configuré pour générer un signal de stimulation après avoir reçu le au moins un signal de commande pour actionner au moins l'une des première et seconde électrodes pour stimuler un nerf à l'intérieur du tissu pincé entre les première et seconde électrodes.

11. Système selon la revendication 10, dans lequel le dispositif intelligent est en communication opérationnelle avec au moins une mémoire et une base de données stockant une pluralité de régimes de traitement correspondant à une pluralité de conditions.

12. Système selon la revendication 10, comprenant en outre un dispositif de surveillance en communication opérationnelle avec au moins l'un du dispositif intelligent et du dispositif de stimulation, dans lequel le dispositif de surveillance transmet des informations de biomarqueur à l'au moins un du dispositif intelligent et du dispositif de stimulation.

13. Système selon la revendication 12, dans lequel le dispositif intelligent détermine au moins une condition d'un patient en utilisant les informations de biomarqueur, et dans lequel le dispositif intelligent détermine au moins un régime de traitement parmi la pluralité de régimes de traitement pour traiter la au moins une condition du patient.

14. Système selon la revendication 10, dans lequel le dispositif intelligent inclut au moins une application ayant une interface utilisateur graphique correspondante pour recevoir au moins une entrée utilisateur pour contrôler au moins un paramètre de fonctionnement du dispositif de stimulation.

15. Dispositif de stimulation selon la revendication 1, dans lequel au moins un des premier et deuxième bras est configuré pour se déplacer entre au moins une configuration pliée et une configuration non pliée.
